# EUROPEAN PATENT SPECIFICATION

(11) **EP 0 507 321 B1**
(45) Date of publication and mention of the grant of the patent: **28.06.1995**
(21) Application number: 92105781.6
(22) Date of filing: 03.04.1992
(51) Int. Cl.: A61M 39/00

(54) **Method and apparatus for joining tubes**
Verfahren und Vorrichtung zur Verbindung von Rohren
Procédé et dispositif pour raccorder des tuyaux

(30) Priority: 05.04.1991 JP 99865/91
(43) Date of publication of application: 07.10.1992
(73) Proprietor: TERUMO KABUSHIKI KAISHA, Tokyo (JP)
(72) Inventor: Nakamura, Atsuo, c/o Terumo Kabushiki Kaisha, Fujinomiya-shi, Shizuoka-ken (JP)
(74) Representative: Casalonga, Axel

(56) References cited:
- EP-A- 0 194 873
- EP-A- 0 238 750
- FR-A- 2 578 782

## Description

The present invention relates to a method of aseptically joining tubes in, for example, a blood bag system or a continuous ambulatory peritoneal dialysis system, culturing system or the like, and to an apparatus for working the particular method.

In a blood bag system, the blood within a first bag (parent bag) is transfused into a second bag (child bag). Also, a treating agent within the second bag is transfused into the first bag. For achieving the transfusion satisfactorily, it is necessary to aseptically join a tube from the first bag to a tube from the second bag. When it comes to a continuous ambulatory peritoneal dialysis system, a dialyzing solution is supplied to the abdominal cavity of a patient and, thus, it is necessary to aseptically join a transfer tube, which is connected to the abdominal cavity, to a bag tube connected to the dialyzing solution bag.

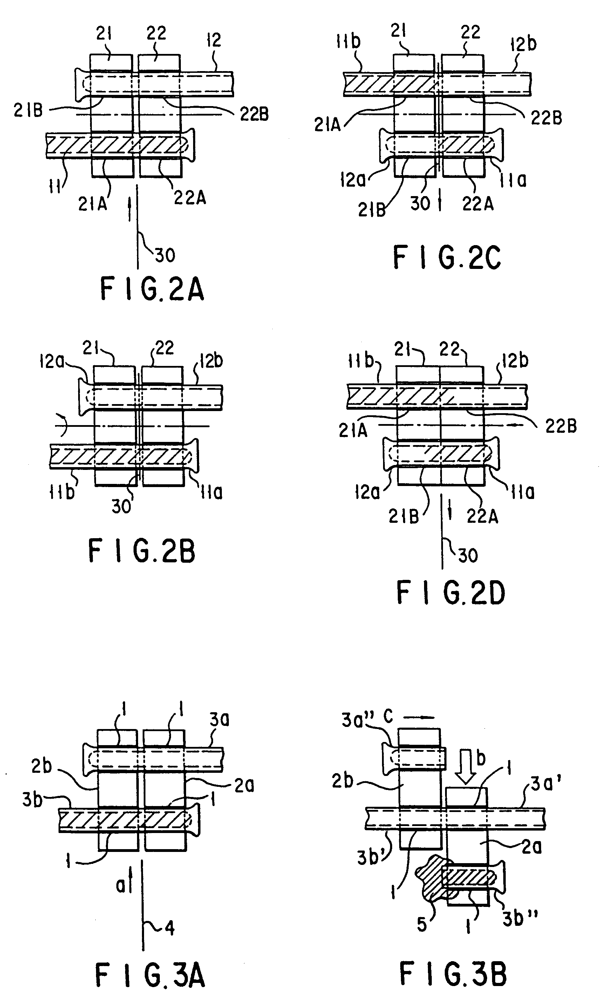

A method of aseptically joining two tubes in the systems described above is disclosed in, for example, Published Examined Japanese Patent Application No. 61-30582, which corresponds to U.S. Patent No. 4,369,779. The conventional method disclosed in this prior art is schematically shown in Figs. 3A and 3B. As shown in Fig. 3A, a pair of tube holders 2a and 2b each having two grooves 1 are arranged such that the grooves 1 of the tube holder 2a are aligned with the grooves 1 of the tube holder 2b. Under this condition, the free end portion of a tube 3a is inserted into the upper grooves 1 extending through the tube holders 2a and 2b. Likewise, the free end portion of a tube 3b is inserted into the lower grooves 1 extending through the tube holders 2a and 2b. As seen from the drawing, these tubes 3a and 3b, which are to be joined, extend in the opposite directions with respect to the free end portions and are fixed under this condition. Then, a cutter blade 4 is moved in a direction denoted by an arrow "a" so as to cut the tubes 3a and 3b at the junction between the tube holders 2a and 2b. The cutting surface of the cutter blade 4 is heated in advance, with the result that the cut surfaces of the tubes 3a and 3b are fused to the cutting surface of the cutter blade 4. It follows that the inner region of each of the tubes 3a and 3b is temporarily kept sealed by the cutting surface of the cutter blade 4.

In the next step, one of the tube holders, e.g., the tube holder 2a, is moved in a direction denoted by an arrow "b" in Fig. 3B so as to permit body portions 3a' and 3b' of the tubes 3a and 3b to be aligned with each other at the cut portions. Under this condition, the cutter blade 4 is withdrawn from between the tube holders 2a and 2b so as to be brought back to the original position. At the same time, one of the tube holders, e.g., the tube holder 2b, is slidden in a direction denoted by an arrow "c" in Fig. 3B and the body portions 3a', 3b' of the tubes 3a, 3b are fused together at the cut surfaces.

The conventional method described above certainly permits aseptically joining two tubes. However, the cut end portions 3a'', 3b'' of the tubes 3a, 3b, which are separated from the tube body portions 3a' and 3b' after the cutting with the cutter blade 4, are elastically expanded after the tube holder 2a is moved in the direction denoted by the arrow "b" as shown in Fig. 3B. As a result, the cut surfaces of these cut end portions 3a'', 3b'' are rendered open. Naturally, the blood or transfusing liquid 5 remaining in, for example, the cut end portion 3b'' flows outside through the open end of the cut end portion 3b'' or is vigorously scattered through the open end, as shown in Fig. 3B. It follows that the working environment or the worker is likely to be contaminated with the humor such as urine or blood, the transfusing liquid, or the continuous ambulatory peritoneal dialysis solution or the like coming out of the cut end portion of the tube.

An object of the present invention is to provide a method of joining two tubes having substantially the same inner diameter or outer diameter while preventing the blood, transfusing liquid or in the tube from coming out of the cut end portions of the tubes to be joined. Another object is to provide an apparatus for working the particular method of the present invention.

According to the present invention, there is provided a method of joining a first thermoplastic tube to a second thermoplastic tube substantially equal in inner diameter or outer diameter to the first tube, comprising the steps of:
(a) cutting free end portions of the first and second thermoplastic tubes with a cutter blade having a heated cutting surface into cut end portions and main tube body portions respectively;
(b) aligning cut end faces of the main tube bodies of the first and second thermoplastic tubes simultaneously with alignment of cut end faces of the cut end portions of the first and second thermoplastic tubes, while keeping cut faces of the first and second thermoplastic tubes closed by the cutter blade; and
(c) withdrawing the cutter blade from the cut faces of the first and second thermoplastic tubes, immediately followed by bringing the cut faces of the main tube bodies of the first and second thermoplastic tubes into mutual tight contact to be welded each other and simultaneously bringing the cut faces of the cut end portions of the first and second thermoplastic tubes into mutual tight contact to be joined each other.

It is desirable to set, before step (a) of the method given above, the free end portions of the first and second thermoplastic tubes over a pair of tube holders, at least one of said tube holders being made rotatable. Also, it is desirable to rotate at least one of the tube holders such that a difference of 180° is provided in the angular position between the two tube holders in step (b) of aligning the cut end faces of the tube bodies of the first and second thermoplastic tubes. Further, it is desirable to collapse, by pushing, a region in the vicinity of the free end portion of each of the first and second thermoplastic tubes so as to close the passages of these tubes before the cutting step (a).

The present invention also provides an apparatus for joining first and second thermoplastic tubes substantially equal to each other in the inner diameter or outer diameter, comprising:
a pair of tube holders positioned adjacent to each other for holding those portions of the first and second thermoplastic tubes at which these first and second tubes are to be joined to each other;
a cutter blade disposed to be movable through the clearance between the two tube holders for cutting those portions of the first and second thermoplastic tubes at which these first and second tubes are to be joined to each other;
heating means for heating the faces of the cutter blade;
a cutter blade driving device for moving the cutter blade through the clearance between the two tube holders so as to actuate the cutter blade to cut the first and second thermoplastic tubes into cut end portions and main tube body portion respectively;
and
a tube holder moving device for allowing the pair of the tube holders to be brought into mutual contact; characterized by
a tube holder rotating device for rotating at least one of the pair of the tube holders at an angle of at least 90° so as to allow a cut surface of the main tube body portion of the first thermoplastic tube to be aligned with a cut surface of the main tube body portion of the second thermoplastic tube and to allow a cut surface of the cut end portion of the first thermoplastic tube to be aligned with a cut surface of the cut end portion of the second thermoplastic tube.

Each of the two tube holders may consist of a split mold provided with grooves accepting insertion of the first and second thermoplastic tubes. The split mold may be of the construction that, when the upper mold half is combined with the lower mold half, the grooves formed in the split mold press and collapse those portions of the first and second thermoplastic tube at which these first and second tubes are to be joined to each other.

This invention can be more fully understood from the following detailed description when taken in conjunction with the accompanying drawings, in which:
Fig. 1 is an oblique view showing a tube joining apparatus according to one embodiment of the present invention;
Figs. 2A to 2D are plan views collectively showing a method of joining two tubes by using the apparatus shown in Fig. 1; and
Figs. 3A and 3B are plan views showing a conventional method of joining tubes.

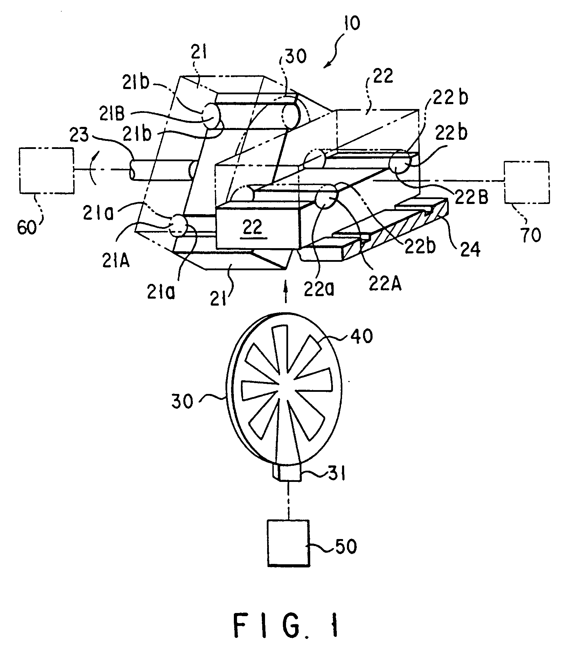

Figs. 1 and 2A to 2D collectively show a method and apparatus for joining two tubes according to the technique of the present invention. Specifically, Fig. 1 shows a tube joining apparatus 10. As seen from the drawing, the tube joining apparatus 10 comprises a pair of tube holders, i.e., a rotatable holder 21 and a pressing holder 22, a cutter blade 30, a heating body 40, a cutter blade driving device 50, a tube holder rotating device 60, and a tube holder moving device 70.

The cutter blade 30 is made of the metal such as copper, aluminum, or the alloy thereof. The heating body 40 is made of the electrical resistance type heating member such as stainless steel or Ni-Cr alloy, and the shape of the heating body 40 is wire type or film type.

Each of the rotatable holder 21 and the pressing holder 22 consists of a split mold. In the drawing, the upper halves of the split molds are denoted by imaginary lines, with the lower mold halves being denoted by solid lines. Two parallel grooves 21a, 21b are formed at the interface between the upper and lower mold halves of the rotatable holder 21. Likewise, two parallel grooves 22a, 22b are formed at the interface between the upper and lower mold halves of the pressing holder 22. These grooves act as tube guiding holes 21A, 21B, 22A and 22B, respectively. To be more specific, the tube guiding hole 21A formed in the rotatable holder 21 is aligned with the tube guiding hole 22A formed in the pressing holder 22. On the other hand, the tube guiding hole 21B formed in the rotatable holder 21 is aligned with the tube guiding hole 22B formed in the pressing holder 22. It should be noted that the grooves 21a, 21b, 22a and 22b are formed shallower and wider in the interfacial region between the rotatable holder 21 and the pressing holder 22. When the upper mold halves are combined with the lower mold halves so as to form the tube guiding holes, the thermoplastic tubes positioned within the tube guiding holes are pressed and collapsed such that the mutually facing regions of the tube are brought into mutual contact so as to allow the tube to be flattened.

One end of a rotary shaft 31 is connected to the tube holder rotating device, with the other end being connected to the rotatable holder 21. When the rotatable holder 21 is rotated by 180° from the original position by the tube holder rotating device 60, the tube guiding hole of the rotatable holder 21, which is originally aligned with one of the tube guiding holes of the pressing holder 22, is aligned with the other tube guiding hole of the pressing holder 22. To be more specific, the tube guiding hole 21A of the rotatable holder 21 is aligned with the tube guiding hole 22B of the pressing holder 22 after rotation of the rotatable holder by 180°. Likewise, the tube guiding hole 21B is aligned with the tube guiding hole 22A.

The pressing holder 22 is movable along a guide mechanism 24 toward and away from the rotatable holder 21. The reciprocating movement of the pressing holder 22 is performed by driving the tube holder moving device 70.

The cutter blade 30 is preferably in the form of a thin disk (about 0.2 to 1.0 mm thickness) having cutting edges formed along the substantially circumferential periphery. A heating body 40 consisting of an electrical resistance type heating device is housed in the cutter blade 30 with the adhesive such as epory type or acryl type, with the result that the cut surfaces of the tubes are heated. The heating body 40 is preferably arranged in a radiate manner with the predetermined angle and distance. The cutter blade 30 is connected to a moving mechanism 31, which is further connected to the cutter blade driving device 50. The cutter blade 30 is reciprocated along the clearance between the rotatable holder 21 and the pressing holder 22, as denoted by broken lines in the drawing.

The tube joining apparatus 10 of the construction described above is used for joining two tubes having substantially the same inner or outer diameter. In joining a thermoplastic tube 11, which is connected to, for example, a first bag, to another tube 12, which is connected to a second bag, these tubes 11 and 12 are inserted into and fixed within the tube guiding holes, as shown in Fig. 2A. To be more specific, the tube 11 is inserted through a hole including he tube guiding hole 21A formed in the rotatable holder 21 and the tube guiding hole 22A formed in the pressing holder 22, said tube guiding holes 21A and 22A communicating with each other. On the other hand, the tube 12 is inserted through a hole including the tube guiding holes 21B and 22B communicating with each other. In this step, the joining portions of the tubes 11 and 12 are pressurized by the rotatable holder 21 and the pressing holder 22 so as to be flattened.

In the next step, the cutter blade 30 is inserted through the clearance between the rotatable holder 21 and the pressing holder 22 by operating the cutter blade driving device 50 so as to cut the tubes 11 and 12, as shown in Fig. 2B. As a result, the cut end portions 11a, 12a of the tubes 11, 12 are separated from the main tube body portions 11b, 12b, respectively. As described previously, the cutting edge surfaces of the cutter blade 30 are heated in advance by the heating body 40. It follows that the cut surfaces of the cut end portions 11a, 12a are somewhat melted, with the result that the mutually facing inner walls of these cut end portions are temporarily sealed and, at the same time, tightly adhered to the cutting edge portions of the cutter blade 30. Likewise, the cut surfaces of the main tube body portions 11b, 12b are somewhat melted, with the result that the mutually facing inner walls of these main tube body portions are temporarily sealed and, at the same time, tightly adhered to the cutting edge portions of the cutter blade 30. In this fashion, the edge portion of each tube is kept sealed temporarily. Incidentally, it is not absolutely necessary for the cutting edge surfaces of the cutter blade 30 to act as a sealing member as far as the temporary sealing can be ensured by the bonding of the mutually facing regions of the inner surface of the tube.

The cutting edge is heated up to the temperature enough to melt the tube and sterilize the tube.

In the next step, the rotatable holder 21 is rotated by 180°, with the cutter blade 30 held in the clearance between the rotatable holder 21 and the pressing holder 22 so as to keep the temporary sealing ensured. As a result, the main tube body portions 11b and 12b are aligned with each other, with the cut faces of these main tube body portions being positioned to face each other, as shown in Fig. 2C. In this step, the cut end portions 11a, 12a of the tubes are also aligned with each other such that the cut faces of these cut end portions are positioned to face each other.

After the rotation of the rotatable holder 21, the cutter blade 30 is withdrawn from the clearance between the cut faces of the main tube body portions 11b, 12b and between the cut end portions 11a, 12a of the tubes, followed by immediately operating the tube holder driving device 70 so as to move the pressing holder 22 toward the rotatable holder 21 and, thus, to push the cut face of the main tube body portion 12b against the cut face of the main tube body portion 11b, as shown in Fig. 2D. Since the cut faces of the main tube body portions and the cut end portions of the tubes 11 and 12 are under molten state in this step, the main tube body portions 11b and 12b are welded to join each other. Likewise, the cut end portions 11a and 12a are welded to liquid-tightly join each other.

As described above, the cut surfaces of the cut end portions 11a, 12a of the tubes are welded to join each other in the step of mutually fusing the cut surfaces of the tube body portions 11b, 12b. It follows that it is unnecessary to worry about the difficulty inherent in the prior art, i.e., the problem that the humor such as urine or blood, the peritoneal dialysis solution or the like remaining in the cut end portions comes outside or is scattered.

In the embodiment described above, the grooves 21a, 21b, 22a, and 22b in the rotatable holder 21 and the pressing holder 22 are formed shallower and wider in the interfacial region between the rotatable holder 21 and the pressing holder 22. In addition, the tubes inserted into these grooves are pressurized and collapsed when the upper mold halves of the rotatable holder and the pressing holder are engaged with the lower mold halves of these holders. In other words, the mutually facing regions of the inner surfaces of these tubes are brought into mutual contact so as to flatten these tubes. However, the present invention is not restricted to this embodiment. For example, the grooves 21a, 21b, 22a, 22b may be circular in cross section such that the tubes inserted thereinto are substantially prevented from being collapsed. In this case, it is necessary to ensure a temporary sealing between the cut surfaces of the tubes 11, 12 and the cutting edge surface of the cutter blade 30 during the period ranging between the cutting step of the tubes 11, 12 with the cutter blade 30 and the aligning step after rotation of the rotatable holder 21 by 180°, i.e., the aligning step between the cut surfaces of the main tube body portions 11b, 12b and between the cut surfaces of the cut end portions 11a, 12a.

It should also be noted that, in the embodiment described above, the rotatable holder 21 alone is rotated by 180°. Needless to say, however, it is also possible to rotate both the rotatable holder 21 and the pressing holder 22, provided that the relative rotation of 180° is achieved between these holders.

Further, in the embodiment described above, the pressing holder 22 alone is moved toward the rotatable holder 21. However, it is also possible to permit both the rotatable holder 21 and the pressing holder 22 to be moved toward each other.

Still further, in the embodiment described above, the cutter blade 30 is in the form of a thin disk having cutting edges formed along the outer circumferential region. However, it is of course possible for the cutter blade 30 to be of any optional shape.

As described above in detail, according the present invention, the cut surfaces of the cut end portions of the tubes are fused to each other during the step of welding the cut surfaces of the main tube body portions. It follows that it is unnecessary to worry about the difficulty inherent in the prior art, i.e., the difficulty that the humor such as urine or blood, the peritoneal dialysis solution or the like remaining in the cut end portions of the tubes comes outside or is scattered so as to stain or infect the working environment or the worker.

In the case of the peritoneal dialysis system, the patient can be prevented from the infections caused by the bacteria, virus or the like.

## Claims

1. A method of joining a first thermoplastic tube to a second thermoplastic tube substantially equal in inner diameter or outer diameter to the first thermoplastic tube, comprising the steps of:
(a) cutting free end portions of the first and second thermoplastic tubes with a cutter blade having a heated cutting edge surfaces into cut end portions and main tube body portions respectively;
(b) aligning cut end faces of the main tube bodies of the first and second thermoplastic tubes simultaneously with alignment of cut end faces of the cut end portions of the first and second thermoplastic tubes, while keeping cut faces of the first and second thermoplastic tubes closed by the cutter blade; and
(c) withdrawing the cutter blade from the cut faces of the first and second thermoplastic tubes, followed by immediately bringing the cut faces of the main tube bodies of the first and second thermoplastic tubes into mutual tight contact to be welded each other and simultaneously bringing the cut faces of the cut end portions of the first and second thermoplastic tubes into mutual tight contact to be joined each other.

2. The method of joining the first and second thermoplastic tubes according to claim 1, wherein, before step (a), the free end portions of the first and second thermoplastic tubes are positioned over a pair of tube holders, at least one of said tube holders being made rotatable, and at least one of the tube holders is rotated by 180° in step (b).

3. The method of joining the first and second thermoplastic tubes according to claim 2, wherein only one of said pair of tube holders is made rotatable by 180°.

4. The method of joining the first and second thermoplastic tubes according to claim 2, wherein, before step (a), the free end portions of the first and second thermoplastic tubes are collapsed by pushing such that these thermoplastic tubes are substantially flattened and fixed to the tube holders.

5. The method of joining the first and second thermoplastic tubes according to claim 1, wherein, before step (a), the free end portions of the fist and second thermoplastic tubes are sealed in advance in the end portions.

6. An apparatus for joining first and second thermoplastic tubes substantially equal to each other in the inner diameter or outer diameter, comprising:
a pair of tube holders (21, 22) positioned adjacent to each other for holding those portions of the first and second thermoplastic tubes at which these first and second tubes are to be joined to each other;
a cutter blade (30) disposed to be movable through the clearance between the two tube holders (21, 22) for cutting those portions of the first and second thermoplastic tubes at which these first and second tubes are to be joined to each other;
heating means (40) for heating the cutting faces of the cutter blade (30);
a cutter blade driving device (50) for moving the cutter blade (30) through the clearance between the two tube holders (21, 22) so as to actuate the cutter blade (30) to cut the first and second thermoplastic tubes into cut end portions and main tube body portion respectively;
and
a tube holder moving device (70) for allowing the pair of the tube holders to be brought into mutual contact; characterized by
a tube holder rotating device (60) for rotating at least one of the pair of the tube holders at an angle of at least 90° so as to allow a cut surface of the main tube body portion of the first thermoplastic tube to be aligned with a cut surface of the main tube body portion of the second thermoplastic tube and to allow a cut surface of the cut end portion of the first thermoplastic tube to be aligned with a cut surface of the cut end portion of the second thermoplastic tube.

7. The apparatus for joining first and second thermoplastic tubes according to claim 6, wherein each of said pair of tube holders (21, 22) is formed of a split mold provided with grooves into which said first and second thermoplastic tubes can be inserted, each of said grooves being shaped such that those portions of the first and second thermoplastic tubes at which these tubes are to be joined are collapsed by the groove (21a, 21b, 22a, 22b) when the upper and lower mold halves are combined to form said split mold.

8. The apparatus for joining first and second thermoplastic tubes according to claim 6, wherein said tube holder rotating device (60) permits rotating only one of said pair of tube holders by 180°.

9. The apparatus for joining first and second thermoplastic tubes according to claim 6, wherein said tube holder moving device (70) permits moving only one of said pair of tube holders.

10. The apparatus for joining first and second thermoplastic tubes according to claim 6, wherein said cutting means is in the form of a thin disk.

## Patentansprüche

1. Verfahren zur Verbindung eines ersten thermoplastischen Rohrs mit einem zweiten thermoplastischen Rohr mit im wesentlichen gleichem Innendurchmesser oder Außendurchmesser wie das erste thermoplastische Rohr, umfassend die Schritte, daß:
(a) freie Endabschnitte des ersten und zweiten thermoplastischen Rohrs mit einem Schneidemesser mit erwärmten Schneidkantenflächen in abgeschnittene Endabschnitte und in Hauptrohrkörperabschnitte geschnitten werden;
(b) Schnittstirnseiten der Hauptrohrkörper des ersten und zweiten thermoplastischen Rohrs gleichzeitig mit Ausrichtung von Schnittstirnseiten der abgeschnittenen Endabschnitte des ersten und zweiten thermoplastischen Rohrs ausgerichtet werden, während Schnittseiten des ersten und zweiten thermoplastischen Rohrs durch das Schneidemesser verschlossen gehalten werden; und
(c) das Schneidemesser von den Schnittseiten des ersten und zweiten thermoplastischen Rohrs zurückgezogen wird, gefolgt davon, daß die Schnittseiten der Hauptrohrkörper des ersten und zweiten thermoplastischen Rohrs sofort in wechselweisen festen Kontakt für das Verschweißen miteinander gebracht werden und gleichzeitig die Schnittseiten der abgeschnittenen Endabschnitte des ersten und zweiten thermoplastischen Rohrs in wechselweisen festen Kontakt gebracht werden, um miteinander verbunden zu werden.

2. Verfahren zur Verbindung des ersten und zweiten thermoplastischen Rohrs nach Anspruch 1, bei dem vor dem Schritt (a) die freien Endabschnitte des ersten und zweiten thermoplastischen Rohrs über einem Paar von Rohrhaltern angeordnet werden, wobei wenigstens einer der Rohrhalter drehbar gemacht ist, und wenigstens einer der Rohrhalter beim Schritt (b) um 180° gedreht wird.

3. Verfahren zur Verbindung des ersten und zweiten thermoplastischen Rohrs nach Anspruch 2, bei dem lediglich einer von dem Paar von Rohrhaltern um 180° drehbar gemacht ist.

4. Verfahren zur Verbindung des ersten und zweiten thermoplastischen Rohrs nach Anspruch 2, bei dem vor dem Schritt (a) die freien Endabschnitte des ersten und zweiten thermoplastischen Rohrs zusammengedrückt werden, indem derart geschoben wird, daß diese thermoplastischen Rohre im wesentlichen abgeflacht und an den Rohrhaltern befestigt werden.

5. Verfahren zur Verbindung des ersten und zweiten thermoplastischen Rohrs nach Anspruch 1, bei dem vor dem Schritt (a) die freien Endabschnitte des ersten und zweiten thermoplastischen Rohrs vorab in den Endabschnitten abgedichtet werden.

6. Vorrichtung zur Verbindung erster und zweiter thermoplastischer Rohre mit im wesentlichen gleichem Innendurchmesser oder Außendurchmesser, umfassend:
- ein Paar von Rohrhaltern (21, 22), die einander benachbart angeordnet sind, um diese Abschnitte des ersten und zweiten thermoplastischen Rohrs zu halten, bei denen diese ersten und zweiten Rohre miteinander verbunden werden sollen;
- ein Schneidemesser (30), das vorgesehen ist, so daß es durch den Zwischenraum zwischen den beiden Rohrhaltern (21, 22) bewegbar ist, um diejenigen Abschnitte des ersten und Zweiten thermoplastischen Rohrs zu schneiden, bei denen diese ersten und zweiten Rohre miteinander verbunden werden sollen;
- eine Heizeinrichtung (40) zum Erwärmen der Schneidseiten des Schneidemessers (30);
- eine Schneidemesserantriebseinrichtung (50) zum Bewegen des Schneidemessers (30) durch den Zwischenraum zwischen den beiden Rohrhaltern (21, 22), um das Schneidemesser (30) zum Schneiden des ersten und zweiten thermoplastischen Rohrs in abgeschnittene Endabschnitte und einen Hauptrohrkörperabschnitt jeweils zu betätigen; und
- eine Rohrhalterbewegungseinrichtung (70), um es zu ermöglichen, daß das Paar der Rohrhalter in wechselseitigen Kontakt gebracht wird;
**gekennzeichnet** durch eine Rohrhalterdreheinrichtung (60) zum Drehen wenigstens einen des Paars der Rohrhalter in einem Winkel von wenigstens 90°, um es zu ermöglichen, daß eine Schneidfläche des Hauptrohrkörperabschnittes des ersten thermoplastischen Rohrs mit einer Schneidfläche des Hauptrohrkörperabschnittes des zweiten thermoplastischen Rohrs ausgerichtet wird, und es zu ermöglichen, daß eine Schneidfläche des abgeschnittenen Endabschnittes des ersten thermoplastischen Rohrs mit einer Schneidfläche des abgeschnittenen Endabschnittes des zweiten thermoplastischen Rohrs ausgerichtet wird.

7. Vorrichtung zur Verbindung eines ersten und zweiten thermoplastischen Rohrs nach Anspruch 6, bei der jeder aus dem Paar der Rohrhalter (21, 22) aus einer geteilten Form gebildet ist, versehen mit Nuten, in die die ersten und zweiten thermoplastischen Rohre eingesetzt werden können, wobei jede der Nuten so geformt ist, daß diejenigen Abschnitte des ersten und zweiten thermoplastischen Rohrs, bei denen diese Rohre verbunden werden sollen, durch die Nut (21a, 21b, 22a, 22b) zusammengedrückt werden, wenn die obere und untere Formhälfte zur Bildung der geteilten Form kombiniert werden.

8. Vorrichtung zur Verbindung eines ersten und zweiten thermoplastischen Rohrs nach Anspruch 6, bei der die Rohrhalterdreheinrichtung (60) es gestattet, lediglich einen aus dem Paar der Rohrhalter um 180° zu drehen.

9. Vorrichtung zur Verbindung eines ersten und zweiten thermoplastischen Rohrs nach Anspruch 6, bei dem die Rohrhalterbewegungseinrichtung (70) es gestattet, lediglich einen aus dem Paar von Rohrhaltern zu bewegen.

10. Vorrichtung zur Verbindung eines ersten und zweiten thermoplastischen Rohrs nach Anspruch 6, bei der die Schneideeinrichtung in der Form einer dünnen Scheibe ist.

## Revendications

1. Procédé pour raccorder un premier tube en matière thermoplastique à un second tube en matière thermoplastique ayant un diamètre intérieur ou un diamètre extérieur sensiblement égal à celui du premier tube thermoplastique, procédé qui comprend les étapes consistant à :
(a) couper des parties d'extrémités libres des premier et second tubes thermoplastiques avec une lame coupante ayant une surface de coupe chauffée pour donner respectivement des parties formant extrémités coupées et des parties formant corps de tube principaux;
(b) aligner les faces d'extrémités coupées des corps de tubes principaux des premier et second tubes thermoplastiques en même temps qu'on aligne les faces d'extrémité coupées des parties formant extrémités coupées des premier et second tubes thermoplastiques tout en maintenant les faces coupées des premier et second tubes thermoplastiques fermées par la lame coupante; et
(c) retirer la lame coupante des faces coupées des premier et second tubes thermoplastiques, puis amener immédiatement les faces coupées des corps de tube principaux des premier et second tubes thermoplastiques en contact mutuel étroit pour être soudées l'une à l'autre et amener simultanément les faces coupées des parties formant extrémités coupées des premier et second tubes thermoplastiques en contact mutuel étroit pour être réunies l'une à l'autre.

2. Procédé pour raccorder des premier et second tubes thermoplastiques selon la revendication 1, dans lequel, avant l'étape (a), les parties d'extrémités libres des premier et second tubes thermoplastiques sont placées sur une paire de supports de tube, l'un au moins desdits supports de tube pouvant tourner et l'un au moins desdits supports de tube tournant de 180° à l'étape (b).

3. Procédé pour raccorder des premier et second tubes thermoplastiques selon la revendication 2, dans lequel un seul des supports de tube de ladite paire peut tourner de 180°.

4. Procédé pour raccorder des premier et second tubes thermoplastiques selon la revendication 2, dans lequel, avant l'étape (a), les parties d'extrémités libres des premier et second tubes thermoplastiques sont écrasées par poussée, de telle sorte que ces tubes thermoplastiques sont substantiellement aplatis et fixés aux supports de tube.

5. Procédé pour raccorder des premier et second tubes thermoplastiques selon la revendication 1, dans lequel, avant l'étape (a), les parties d'extrémités libres des premier et second tubes thermoplastiques sont scellées à l'avance dans les parties d'extrémités.

6. Dispositif pour raccorder des premier et second tubes en matière thermoplastique ayant un diamètre intérieur ou un diamètre extérieur sensiblement égal, qui comprend :
- une paire de supports de tube (21,22) placés adajacents l'un à l'autre pour maintenir les parties des premier et second tubes thermoplastiques au niveau desquelles ces premier et second tubes doivent être réunis l'un à l'autre;
- une lame coupante (30) placée pour se déplacer à travers le jeu existant entre les deux supports de tube (21,22), afin de couper les parties des premier et second tubes thermoplastiques au niveau desquelles les premier et second tubes doivent être réunis l'un à l'autre;
- un moyen de chauffage (40) pour chauffer les faces coupantes de la lame coupante (30);
- un dispositif (50) d'entraînement de lame coupante pour déplacer la lame coupante (30) à travers le jeu compris entre les deux supports de tube (21,22), de manière à amener la lame coupante (30) à couper les premier et second tubes thermoplastiques en des parties formant extrémités coupées et des parties formant corps de tube principaux; et
- un dispositif (70) de déplacement de supports de tube pour permettre à la paire de supports de tube d'être amenée en contact mutuel,
caractérisé par un dispositif (60) de rotation de supports de tube destiné à faire tourner l'un au moins des supports de tube de ladite paire d'un angle d'au moins 90° pour permettre à la surface coupée de la partie formant corps principal de tube du premier tube thermoplastique d'être alignée avec la surface coupée de la partie formant corps principal de tube du second tube thermoplastique et pour permettre à la surface coupée de la partie formant extrémité coupée du premier tube thermoplastique d'être alignée avec la surface coupée de la partie formant extrémité coupée du second tube thermoplastique.

7. Dispositif pour raccorder des premier et second tubes thermoplastiques selon la revendication 6, dans lequel chaque support de tube de ladite paire de supports de tube (21, 22) est formé d'un moule en deux parties muni de rainures dans lesquelles lesdits premier et second tubes thermoplastiques peuvent être introduits, chaque rainure ayant une forme telle que les parties des premier et second tubes thermoplastiques au niveau desquelles ces tubes doivent être réunis, sont écrasées par la rainure (21a, 21b, 22a, 22b) quand les moitiés supérieure et inférieure des moules sont associées pour former ledit moule en deux parties.

8. Dispositif pour raccorder des premier et second tubes thermoplastiques selon la revendication 6, dans lequel ledit dispositif (60) de rotation de support de tube permet de ne faire tourner qu'un seul support de tube de ladite paire de 180°.

9. Dispositif pour raccorder des premier et second tubes thermoplastiques selon la revendication 6, dans lequel ledit dispositif (70) de déplacement de support de tube permet de ne déplacer qu'un seul des supports de tube de ladite paire.

10. Dispositif pour raccorder des premier et second tubes thermoplastiques selon la revendication 6, dans lequel ledit moyen coupant a la forme d'un disque mince.
